Europäisches Patentamt

European Patent Office

Office européen des brevets

(11) Publication number: **0 056 824**
**B1**

## EUROPEAN PATENT SPECIFICATION

(45) Date of publication of patent specification: 02.05.85

(51) Int. Cl.⁴: **A 61 K 35/64**

(21) Application number: **81100434.0**

(22) Date of filing: **22.01.81**

(54) **Therapeutic agent.**

(43) Date of publication of application:
**04.08.82 Bulletin 82/31**

(45) Publication of the grant of the patent:
**02.05.85 Bulletin 85/18**

(84) Designated Contracting States:
**CH DE FR GB LI SE**

(56) References cited:
**US-A-4 247 540**

**THE JOURNAL OF IMMUNOLOGY, vol. 120, no.
5, May 1978 The Williams & Wilkins Co, USA D.
SCHULTZ et al. "Studies of a polysaccharide in
ant venom (Pseudomyrmex sp.) that activates
the classical complement (C) pathway" page
1797, first abstract (Abstracts of papers
presented at the seventh international
complement workshop. St. Petersburg Beach,
Florida, November 20-22, 1977)
THE JOURNAL OF IMMUNOLOGY, vol. 119, no.
5, November 1977 The Williams & Wilkins Co.
USA D. SCHULTZ et al. "Venom of the ant
Pseudomyrmex sp.: Further characterization of
two factors that affect human complement
proteins" pages 1690-1699**

(73) Proprietor: **Holzmann, Gunter**
**Post Office Box 391**
**Santa Cruz (BO)**

(72) Inventor: **Holzmann, Gunter**
**Post Office Box 391**
**Santa Cruz (BO)**

(74) Representative: **Berkenfeld, Helmut, Dipl.-Ing.**
**An der Schanz 2**
**D-5000 Köln 60 (DE)**

**Description**

Background of the invention

This invention relates to a composition and method for relieving the symptoms of such auto-immune ailments as rheumatoid arthritis. The nature of rheumatoid arthritis is such that one familiar with it would be led to conclude that other auto-immune ailments which also involve symptomatic inflammation of tissues will also respond to treatment using the products according to the present invention. Other such auto-immune ailments include asthma, multiple sclerosis (nerve tissue), lupus erythematosus (kidneys), sclerodermata (skin), arteritis, and other somewhat rare ailments classified as connective tissue diseases, or immune complex diseases.

There are a number of theories for the etiology of rheumatoid arthritis including the probability that it is an auto-immunological ailment which requires the existance of an antibody or RA factor in the blood. (Note that RA and RF are used interchangeably herein). The presence of the RA factor antibody is well known and widely used as a confirming test for arthritis. A rheumatoid arthritis causative agent has not been isolated.

Because the present invention works on a second auto-immune ailment, and in view of certain results of laboratory tests in vitro with components of the human body complement system and tests on at least one patient, it is believed that similar auto-immune ailments will also respond to treatment.

The effect of venom factors from the ant *Pseudomyrmex sp.* on human complement proteins and the use of the ant venom in the treatment of rheumatoid arthritis has been described in "The Journal of Immunology" Vol. 119 (1977) pp. 1690—8. In respect of information which would enable the products to be prepared the document is however incomplete insofar as the identity of the particular species involved is not disclosed.

The pain and discomfort due to arthritis is a major disabling factor for large numbers of people. Even without actually curing these diseases, a remission of the symptoms would permit many individuals to lead more profitable and more fulfilling lives.

Brief description

In brief, this invention is in the use of an active agent found in an extract of materials from the venom sac of ants of the genes Pseudomyrmex species triplarinus.

The extract is obtained by crushing the abdomen of the ant. The crushed abdomen are then mixed with an aqueous solvent and the resulting solution filtered, purified, and adjusted to provide a concentration effective for injection into a patient. The extract containing the therapeutic agent is injected subcutaneously into the patient wherein, apparently without significant side effects, it causes remission of arthritic symptoms and appears to significantly decrease the RA factor (measure of an antibody normally associated with rheumatoid arthritis).

An aqueous saline solution (physiological solution) is normally employed as the carrier as this has been found to be compatable with the extract as well as the human body.

In order to identify the active agent in the extract, the human complement system is used as a test of activity of purified substances. The validity of this method of assaying was confirmed by a number of actual tests on humans and rats.

Description of the preferred embodiments

The ant *Pseudomyrmex triplarinus* is limited to certain parts of tropical South America. It belongs to a highly specialized group of ants all of which are restricted in their nesting habits to trees of the genus *Triplaris* which plant group is limited to the American tropics.

More specifically the ant employed in the tests is the worker (sterile female), genus Pseudomyrmex, species triplarinus; sub-family Pseudomyrmicinae, and family Formicidae.

This sub-family has an unusual evolutionary history. There is no agreement on where it should be placed in the evolution of ants. It clearly belongs to the family Formicidae; which is the ant family. But it is not clear what its ancestral strain is or how its ancestral strain is related to the ancestral strains of other ants.

It is probably fair to say that, within the context of agreeing that these are indeed ants, they appear to be totally unrelated to the rest of the ants. They are unrelated in the sense that some of their habits and in particular their anatomy diverge pronouncedly from the habits and the anatomy of other ants. This is one of the reasons for the conclusion that the venom of the ants of the triplarinus species of ant, is unrelated to the venom of other insects including the venom of any other group of ants.

Extraction of the active agent

Method I (Aqueous extraction)

In one method for making a composition for injecting into a patient, the abdomens are separated from the ants. The abdomen contains the venom sac. A fixed number of separated abdomens are then macerated (stirred, squeezed and crushed) in saline physiological solution to dissolve the soluble parts. The mixture is then filtered, removing all insoluble constituents. The result is a solution of portions of the venom and other abdominal juices which are soluble in physiological solution.

# 0 056 824

More specifically, a presently preferred mode of preparing an effective agent involves the following steps: (1) the dissected abdomens are macerated in a saline physiological solution, (2) the result is filtered through a No. 1 gauge filter and the filter is worked three times with physiological solution, (3) the result is diluted with saline physiological solution to a concentration equivalent to the venom from ten ants in one cubic centimeter of solution, (4) controls are taken for bacteriological and microscopic analysis, and (5) after sterility is proven the solution is stored at one to two degrees Centigrade. The solution can be held in sterile ampoules for use.

Use of active agent for symptomatic relief of rheumatoid arthritis

In a number of early tests, the crushed and filtered abdomen of one hundred ants extracted according to Method I and diluted with physiological solution to provide twenty cubic centimeters (20 c.c.) of mixture was used. The resulting concentration is the content of five venom sacs in one c.c. In an initial series of tests, the injections generally started with an amount of solution containing the venom provided by 0.5 ants. Such an amount is called herein 0.5 UF. Doses were given daily to step the amount up over a period of about ten days to a level where doses are equal to the venom from approximately five to ten ants. At first 0.1 c.c. was injected in the upper arm in order to determine the patient's sensitivity to the venom and to prevent anaphylactic reaction. If no adverse reaction was observed, injections then were administered at regular intervals and increasing doses, starting with 0.5 UF. All injections were administered subcutaneously or intradermically. With good tolerance, doses were given daily for about 10 days, increasing the concentration for the last 5 days to 10 UF daily, according to each individual case. The reaction of the skin to these injections is similar to the reaction of the skin toward a sting from the ant except for the important fact that there is no pain. In particular a little white papilla appears around the injection. After a few minutes, a swelling and bright red coloring occurs. In general, these symptoms are slight and disappear within twenty-four (24) hours. No infection was associated with these injections.

It has been found that a treatment of 10 days with amounts representing the equivalent of 5 to 10 ants (venom sacs or abdomen) during the last 5 days provides substantial relief or disappearance of arthritic symptoms. In an initial series of tests given in Bolivia, relief in many cases appeared to last over a period of years. Furthermore, in each case, no adverse side effects were observed.

Test results for utility

Arthritis was simulated in rats for testing the anti-inflammatory properties of the extracted material. In addition, a carefully constructed double-blind test was made on humans. Both tests are briefly described. One test was limited to human subjects carefully selected so that all had rheumatoid arthritis according to the American Rheumatism Association criteria. Accordingly, it is believed that substantial utility for treatment of rheumatoid arthritis has been demonstrated. It is believed that arthritis of other forms are sufficiently divergent in etiology (non-auto-immune ailments) that no reasonable prediction can be made as to the utility of this invention for treating gouty and other forms of arthritis. However, test on laboratory animals indicate that the venom is an anti-inflammatory agent.

Human study I (Symptomatic relief indicia)

To test the potential efficacy of this venom a double-blind clinical trial was undertaken at the Laboratorio Clinico Serrales in Santo Domingo, D. R. This trial was designed by Charles Sisk, M.D., former Director of Medical and Scientific Affaires of the National Arthritic Foundation, and at the time of the test, Associate Professor of Medicine, University of Missouri Medical Center. The principal co-investigator of the study was Harvey Brown, M.D., Professor of Medicine, University of Miami School of Medicine. The trial was conducted during the interval from April 1975 through August 1975.

A method I extract of venom was used. Venom was extracted manually from abdominal pouches of the insect, mixed with a physiological saline solution, refrigerated with ice, and shipped to the Dominican Republic of Bolivia. No attempt was made to further purify the extract as temperature stability and other physiochemical characteristics were not well defined. It is believed that early attempts to add preservatives to the venom may have resulted in causing the venom mixture to become inactive.

Selection of subjects—1

Thirty subjects were located who all met the United States Food and Drug Administration (FDA) definition (1970) of having active rheumatoid arthritis. The FDA definition requires having greater than a defined level of at least three out of four RA parameters as defined by the American Rheumatism Association (ARA) criteria (1958) for rheumatoid arthritis (RA). The four parameters are listed below. The technique for measuring these parameters and the criteria for meeting the FDA definition of RA disease as set forth in FDA and ARA standards was followed.

Prior to study entry all subjects on anti-inflammatory medications were instructed to stop this medication for at least three days before beginning the trial. One criteria established before accepting a subject was that the subject could not be on steroid treatment of over 10 mg. prednison-equivalent during the three-month interval before study entry. No subject had received gold salt treatment before or during the trial.

All subjects were initially randomized to venom-treated and placebo groups in accordance with a

3

double-blind, parallel design. Ten (10) subjects initially randomized to the placebo treatment were subsequently treated with venom thus permitting a crossover comparison of this group.

Procedure—I

Both venom and placebo were administered by subcutaneous injection of the upper arm or thigh. Venom was given in increasing single daily doses over several days until a maximum 1 milliliter (ml) dose was achieved according to the following schedule:

| Day | Dose |
| --- | --- |
| 1 | 0.1 ml |
| 2 | 0.2 ml |
| 3 | 0.3 ml |
| 4 | 0.5 ml |
| 5—14 | 1.0 ml each day |

All injections were administered by laboratory personnel not involved in the evaluation of therapeutic response. Subjects were required to come to the Clinic each day for their injections. Compliance was excellent and no subject deviated from the treatment schedule.

To simulate both the local and systematic effects of the venom, histamine phosphate (0.1 mg. base-equivalent) was given to the placebo group. Although this preparation did not satisfactorily simulate venom effects, precautions were taken to maintain double blindness by covering injection sites during evaluation and by not advising subjects of the types of side effects they might experience from venom or placebo.

All pre-treatment evaluations were performed by one of the co-investigators with three exceptions. The three exceptions were examined by a third rheumatologist trained in the United States. All follow-up evaluations were done by one of the co-investigators.

If patients needed supplementary medication during the study they were given propoxphene and instructed to take it on an as needed basis. They were advised to avoid all other anti-rheumatic medication including aspirin® and any other aspirin-containing medication.

The four parameters used to determine the existence of active RA were also used to evaluate.
   a. number of clinically active joints;
   b. erythrocyte sedimentation rate (ESR), as measured by the Westergren method;
   c. duration of morning stiffness; and
   d. grip strength.
Each of the above four parameters is measured as set forth in the ARA Cooperating Clinics Committee Observer's Guide (1958).

The age range of the 16 cases receiving venom was 35 to 71 years with a mean of 58 years and of the 14 cases in the placebo group 34 to 74 years with a mean of 54 years. In the venom treated group there were 14 females and 2 males, in the placebo group, 7 females and 7 males. Functional classification of these therapeutic groups is shown in Table I. (Infra page 11).

Results—Procedure I

All rheumatological evaluations were done within the week prior to study entry (Pre-Rx), at the termination of the two-week treatment interval (Post-Rx I) and at an interval thereafter (Post-Rx II) which was four weeks after Post-Rx I in the case of some subjects and six weeks in the case of other subjects. The Post-Rx II data, whether taken at four or six weeks after treatment, is combined in the following analysis. The data is summarized in Table II for the parallel venom and placebo groups. Table III is extracted from Table II and provides a summary of the significantly improved parameters. The data indicates that statistically significant improvements occur in grip strength and in the number of clinically active joints in the venom treated group while corresponding improvement does not occur within the placebo treated group.

No adverse reactions other than local ones were observed in subjects receiving venom. All subjects received a battery of laboratory tests including tests for toxicity. These included, pre- and post-treatment CBC, urinalysis, clinical chemistries (SMA-12). None of these parameters suggested any hematologic, renal, hepatic or other toxicity. Local reactions consisted of marked induration, heat and erythema at the points of injection. Most of these reactions subsided spontaneously in one or two weeks after discontinuing treatment.

One subject developed a localized abscess. This responded promptly to antibiotic therapy.

To confirm that the therapeutic agent of this invention did not obtain its therapeutic properties by acting as a stress agent and stimulating endogenous cortisol product, pre- and post-treatment serum cortisol levels were measured in all cases. No differences of before and after therapy hormone level were observed. This result indicates that the dangerous condition known as "adrenal crisis", which is caused by repeated stimulation of the adrenal glands, is not likely to occur as a result of extended treatment according to the present invention.

The mean values of the various rheumatological parameters measured during the trial are set forth in Table II. The statistically significant mean values together with their standard deviations are set forth in Table III.

It might be noted that the improvement obtained was not obtained immediately at the termination of the injections or, at least, the improvement obtained by that time was not statistically significant. The statistically significant improvement was observed at the end of a four or six week period (Post-Rx II) after termination of the injections. Apparently, it takes time for the inflammation to subside and for the body to adjust to the change. It is not yet known for how long the treatment is effective or, more specifically, what the relationship is between time after treatment and level of return of symptoms to pre-treatment levels.

The extent of improvement in the number of clinically active joints is a measure of the number of joints which at Pre-Rx were degrees, 2, 3 or 4 on the ARA definition of degree of involvement of clinically active joints and which became grades zero or one at Post-Rx II. Thus the improvement had to pass over the threshold between degrees 2 and degree 1 in order for it to be recorded as an improvement in the data set forth in the tables. A joint which improved from a fourth degree level (severe) to the second degree level (minimal—definitely present, but mild) under the ARA definitions would not be reflected as an improvement in the data in the tables. It is believed that the extent of the improvement obtained by this treatment and its statistical significance would be increased if the analysis took into account all improvement and not just the improvement that passed the threshold between degrees two and one.

With respect to Table II, it should be noted that the footnoted P levels were made with the appropriate parametric "t" tests for grip strength and for number of clinically active joints.

Ten of the fourteen individuals in the placebo group were given the venom treatment after termination of the parallel venom and placebo trial. The individuals selected were not selected at random but were those who tended to have the worst symptoms. Thus the results of this crossover trial group cannot be rigorously statistically analyzed. Nonetheless, there was a reduction of number of clinically active joints in the groups of ten subjects from a Pre-Rx mean of 23.9 (std. dev. 12.9) to a post treatment mean of 13.0 (std. dev. 11.9). This suggests a statistically significant difference at the P .05 level. The other rheumatological parameters of this cross-over group did not show a statistically significant degree of improvement.

TABLE I

| ARA functional class* | Number in class | |
| --- | --- | --- |
| | Venom group | Placebo group |
| 1 (No handicap) | 0 | 0 |
| 2 (discomfort or limited motion) | 5 | 7 |
| 3 (limited self care) | 8 | 5 |
| 4 (incapacitated) | 3 | 2 |
| | 16 | 14 |

*The class definitions are set out more fully in the Observer's Guide issued by the ARA Cooperating Clinic's Committee.

TABLE II

Mean values of rheumatologic parameter measurements for venom-treated and placebo groups

| Group | Parameter | Mean measurement value | | |
|---|---|---|---|---|
| | | Pre-Rx[a] | Post-RxI[b] | Post-RxII[c] |
| | Grip strength: (mm of Hg) Right Hand | 92.0 | 104.0 | 177.0 |
| | Left Hand | 63.8 | 74.3 | 181.0 |
| "VENOM" (N=16) | Morning stiffness (duration in hours) | 2.8 | 2.5 | 2.2 |
| | E.S.R; Westergren Method (mms of sed. per hr.) | 56.9 | 65.4 | * |
| | Active joints[d] (number of) | 19.6 | 17.3 | 8.6 |
| | Grip strength: (mm of Hg) Right Hand | 99.5 | 103.7 | 102.3 |
| | Left Hand | 105.6 | 114.2 | 125.3 |
| "PLACEBO" (N−14) | Morning stiffness (duration in hours) | 3.4 | 3.1 | 3.0 |
| | E.S.R: Westergren Method (mms of sed. per hr.) | 63.1 | * | * |
| | Active joints[d] (number of) | 24.0 | 19.5 | 15.4 |

*Insufficient Data
[a]·data taken within the week prior to study entry
[b]·data taken at termination of the two week treatment interval
[c]·data taken at four or six weeks after termination of treatment
[d]·ARA criteria for degrees of involvement were used; specifically finger joints meeting the ARA definition for degrees 2, 3 or 4 were considered clinically active. The values listed in the Table are the mean number of clinically active joints for the group.

TABLE III
Statistically significant RA parameter measurements

| Group | Parameter | Pre-Rx mean & std. dev. | Post-Rx II mean & std. dev. |
|---|---|---|---|
| | Grip strength: | | |
| | Right Hand[c] | 92.0 (56.9)[a] | 177.0 (56.6)[b] |
| | Left Hand[d] | 63.8 (26.8)[a] | 181.0 (48.7)[b] |
| "VENOM" (N=16) | | | |
| | Active joints[g] | 19.6 (10.7)[e] | 8.6 (5.0)[f] |
| | Grip strength: | | |
| | Right Hand | 99.5 (51.6)[a] | 102.3 (58.0)[b] |
| | Left Hand | 105.6 (62.8)[a] | 125.3 (58.4)[b] |
| "PLACEBO" | | | |
| | Active joints | 24.0 (13.8)[e] | 15.4 (19.6)[f] |

[a] Grip strength: No significant difference between the means of the venom and placebo groups, Pre-Tx.

[b] Grip strength: The means of the venom and placebo groups, Post-Rx II, show a statistically significant difference; specifically a difference that could arise by chance less often than once in twenty trials (P at .05 level).

[c] Right hand grip strength in the venom group alone; the means of Pre-Rx and Post-Rx II values show a statistically significant difference; i.e. a difference that could arise by chance less often once in one hundred trials (P at .01 level).

[d] Left hand grip strength in the venom group alone: The means of Pre-Rx and Post-Rx II values shows a statistically significant difference; i.e. a difference that could arise by chance less often than once in two hundred trials (P at .005 level).

[e] Number of clinically active joints: No significant difference between the means of the venom and placebo groups, Pre-Rx.

[f] Number of clinically active joints: The means of the venom and placebo groups, Post-Rx II, show a statistically significant difference; specifically a difference that could arise by chance less often than once in twenty trials (P at .05 level).

[g] Number of clinically active joints in the venom group alone: the means of Pre-Rx and Post-Rx II values show a statistically significant difference; i.e. a difference that could arise by chance less often than once in one hundred trials (P at .01 level).

Utility as an anti-inflammatory agent for simulated rheumatoid arthritis in rats

Test were made utilizing rats to show the general anti-inflammatory properties of the subject invention extract and methods for further purifying or using said extract to enhance its effectiveness.

Rheumatoid arthritis was simulated in Sprague-Dolly rats by injecting an adjuvant, Parrigan-C, into the right foot pad of the rat. Measurements were made along the dorsal ventral axis in millimeters. The untreated left foot was also measured as a standard.

These test are generally considered to simulate rheumatoid arthritis closely enough for the test results to be an indication of the effectiveness of the material being used for use in human beings. However, the tests in fact generally indicate the anti-inflammatory properties of the material being tested. The results of the tests show that the subject invention extract not only reduces rheumatoid arthritis symptoms but also reduces the measured RA factor in the blood, and apparently accomplishes these results without increasing the cortisol serum levels in the blood.

Purification of polysaccharide of clinical studies
(Method I-A)

Starting with X ml of extract (Method I (above)) (usually 47 ml, 0.275 mg protein/ml): 9 parts of absolute ethanol are slowly added to 1 part of the extract with constant stirring at 0°C, the mixture is stirred for 1 hr. at 0°C, and centrifuged (Sorval RC2-B, 12,000×g, 30 min, 4°C). The supernatant fluid is discarded, and the precipitate is washed one time with 90 per cent ethanol at 0°C and centrifuged. A small volume of distilled water is added to suspend the material, and it is lyophilized to eliminate the alcohol. Distilled water is added to dissolve the dry powder, it is centrifuged to eliminate insoluble material, and the supernatant fluid is dialyzed vs. 0.018 M sodium phosphate containing 0.15 M NaCl, pH 7.2 (PBS). This preparation, which is mostly polysaccharide but contains at least one protein as shown by polyacrylamide gel electrophoresis, may be stored in the lyophilized state, and is reconstituted by adding the desired volume of distilled water.

Other methods for further purificution such as (a) Concanavalin a—linked Sepharose Chromatography and (b) ultrafiltration membrane have been successfully used but were not used in the preparation of the material used for this study.

This analysis is of a polysaccharide which appears to be the most important, if not the only active factor in the therapeutic agent of the present invention.

The PS had a heterogenous size in native ant venom. The smallest moiety that activated serum Cl had a MW of *ca*. 3,000 daltons as determined by gel chromatography. The PS was not precipitated by barium salts, showing no apparent sulfate groups. The following sugars und the molar ratios were found by gas chromatography: mannose (7.7), fucose (1.8), N-acetylgalactosamine (1.6), galactose (1.4), glucose (1.0), and N-acetylglucosamine (1.0).

Identification of the active factor

The active factor of the present invention was isolated and the isolated material characterized by electrophoresis separation of components followed by reactions with various known reagents; elemental analysis; IR spectrometry; and solubility; all as set forth below:

The drawings:

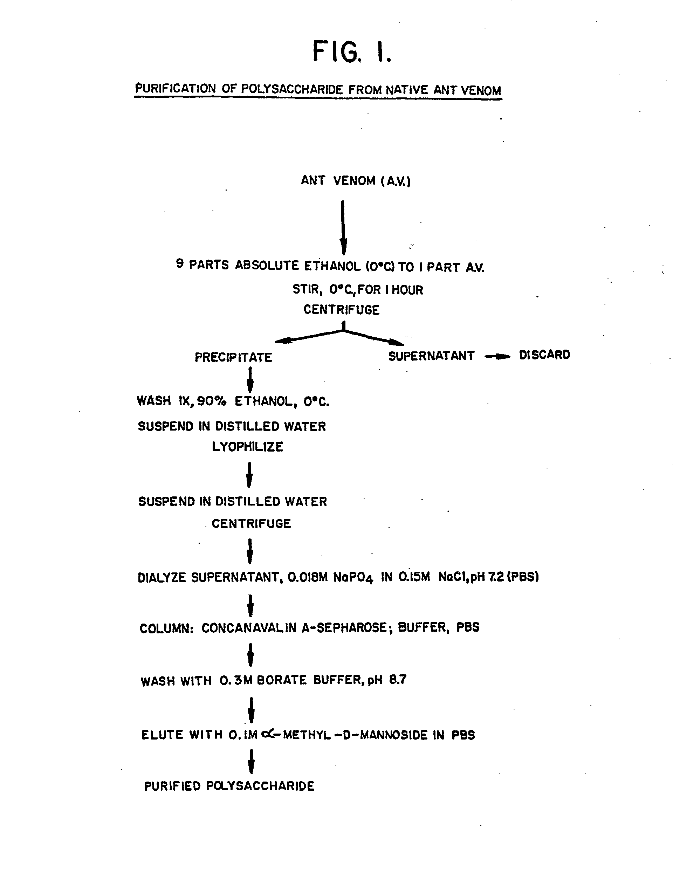

Fig. 1 is a flow diagram for the purification of Polysaccharide from native ant venom (extracted by Method I).

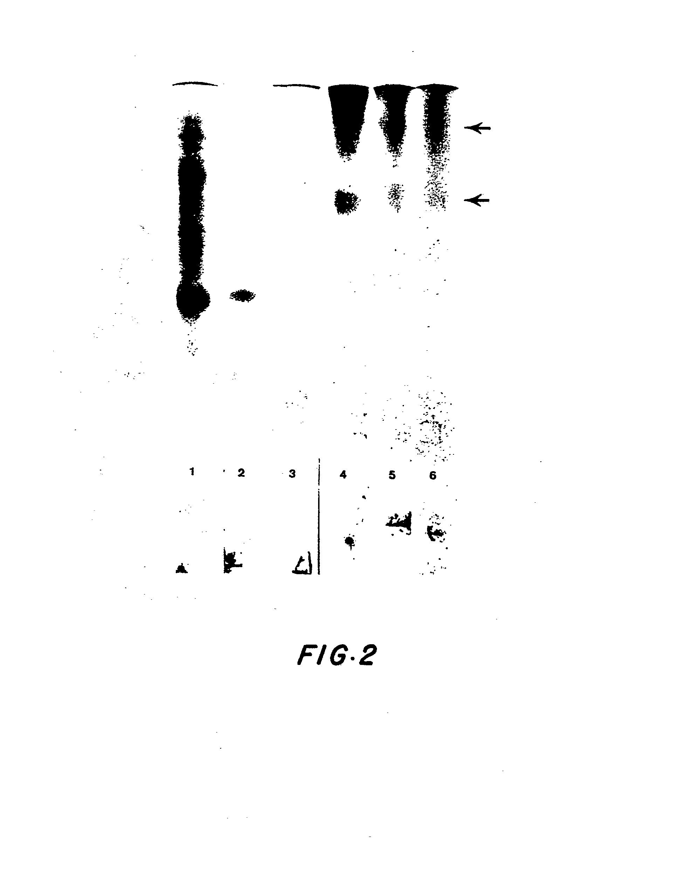

Fig. 2. Analysis of the products of the isolation steps for the ant venom polysaccharide, by disc electrophoresis on polyacrylamide gels. The gels are (1) native ant venom (0.275 mg protein/ml, 180 ng/ml of uronic acid), (2) the alcohol precipitate (lyophilized and solubilized in distilled water, 18 µg/ml of uronic acid), and (3) the product that eluted from the Sepharose-Con A (10.5 µg/ml of uronic acid), all stained for proteins with Amido Black 10B. gel nos. 4, 5, and 6 are the same products as 1, 2, and 3, but stained with periodic acid Schiff reagent. The arrows point to the polysaccharide(s) which caused C1 activation and C4 and C2 consumption in serum.

Fig. 3(A). Elution profile of native ant venom (13.8 mg protein/ml) on Sephadex G-25 at 4°C. Fractions were assayed for protein by absorbance at 280 nm and for sugars with anthrone reagent at 620 nm. Of the 3 anthrone-positive peaks, only I and II caused C4 consumption in normal human serum. Fig. 3(b) shows that peak II eluted at the same position after rechromatography on the same column. Column, 1.1×70 cm; buffer, 5 nM sodium phosphate, 150 nM NaCl, pH 7.5; flow rate, 6 ml/hr.

Fig. 4. Disc electrophoresis of pooled and concentrated fractions within peaks I and II from Fig. 3(A). All gels were stained with periodic acid Schiff (PAS) reagent. Gel no. 1, concentrated peak I; gel no. 2, peak I diluted 1:10, gel no. 3, peak I diluted 1:20; gel no. 4, peak I diluted 1:40; gel no. 5, peak II. The arrows point to PAS-positive bands, and demonstrate the heterogeneity of the polysaccharide.

Fig. 5 is a typical IR Spectra run neat on an alcohol precipitate of ant venom extract.

Fig. 6 is a typical IR Spectra run neat on an a D-mannoside eluate of Con A column of the alcohol precipitate.

Isolation of the polysaccharide (PS)

Nine parts of absolute ethanol were slowly added to 1 part of the venom (47 ml of venom, 0.275 mg protein/ml, 180 µg/ml of uronic acid) with constant stirring at 0°C. A light precipitate formed, the mixture was stirred for 1 hr at 0°C, and centrifuged (Sorvall RC2-B; 12,000×g; 30 min; 4°C). The supernatant fluid was discarded because after evaporation of the alcohol and solubilization in an aqueous buffer, the product did not cause C4 consumption in normal human serum.* The precipitate was washed one time with 90 per cent ethanol at 0°C and centrifuged. A small volume of distilled water was added to suspend the material, and it was lyophilized to eliminate the alcohol. Forty-seven milliliters of distilled water were added to dissolve the dry powder, it was centrifuged to eliminate insoluble material, and the supernatant fluid wus dialyzed against 18 nM sodium phosphate containing 150 mM NaCl, pH 7.2 (PBS). Forty-six milliliters (18 µg/ml of uronic acid) were applied to a 2.4×22 cm column containing Sepharose-Con A that was equilibrated with PBS at 25°C. The column was first washed with PBS, and the latter (300 ml) was concentrated to 46 ml (7 µg/ml of uronic acid) with an Amicon UM-2 membrane (Amicon Corp., Lexington, Mass.). Then the column was thoroughly washed with 300 ml of 300 nM sodium borate buffer, pH 8.7 (no uronic acid recovered), and eluted with 300 ml of 100 mM ∞-methyl-methyl-D-mannoside in PBS. This was concentrated to 46 ml with an Amicon UM-2 membrane, and contained 10.5 µg/ml of uronic acid. All the above pools were concentrated with an Amicon UM-2 membrane to 10 ml at 4°C.

Polyacrylamide gel electrophoresis

The method for polyacrylamide gel electrophoresis using no stacking gel and 7 per cent separating gel

*The polysaccharide caused the activation of the first component of complement (C1) in normal human serum, which in turn acts on its two natural substrates C4 and C2. Thus, C4 and/or C2 consumption indirectly shows that C1 was activated.

(Orstein, 1964) (Davis, 1964) is described in the manual of Canalco Co. (Rockville, MD). Gels were stained for protein with C. I. Acid Black 1, constitution No. 20470 (Amido Black 10B) (Greenfield *et al*, 1971), and for glyco-protein and polysaccharida with periodic acid Schiff reagent (Segrest & Jackson, 1972). Neutral sugars were detected with anthrone reagent (Seifter *et al*, 1950), hexuronic acids by the carbazole method of Bitter and Muir (1962), and phosphate by the method of Ames and Dubin (1960).

Gas chromatography. To anthrone and uronic acid positive samples was added a fine concentration of 3 N HCl, and they were hydrolyzed at 100°C for 3 hr. The hydrolysate were reduced by sodium borohydride, acetylated with acetic anhydride by standard procedures (Griggs *et al*, 1971), and the alditol acetates were analyzed by gas chromatography with a Hewlett-Packard apparatus.

The products of the purification steps were subjected to electrophoresis on polyacrylamide gels, applying 250 µl/gel. In Fig. 2, gel columns no. 1, 2, and 3 are, respectively: native ant venom, the alcohol precipitate that was lyophilized and solubilized in distilled water, and the product that eluted from the Con-A column. They were stained with Amido Black 10B. Gel columns no. 4, 5, and 6 are the same as 1, 2, and 3, but they were stained with periodic acid Schiff reagent (PAS). Most of the venom protein was eliminated by the alcohol precipitation step (gel no. 2). The product that eluted from the Con-A column had no detectable protein (gel. no. 3), but contained material that did not enter the gel, and at least 2 diffuse bands which stained with the PAS (gel no. 6), indicating a polysaccharide(s).

Serial slices of varying thickness from top to bottom (Table 1) of 6 unstained gels containing the Con-A eluate were pooled, eluted 48 hr with 2 ml of DGVB++ at 4°C, and concentrated to 0.5 ml with an Amicon UM-2 membrane at 4°C. Equal volumes of each concentrated eluate and normal human serum were incubated at 37°C for 4 hr, and the C4 levels were determined by functional hemolytic assays. Table 1 shows that the product that consumed serum C4 (i.e., activated C1) was in the top 1 to 3 mm, including a portion that did not enter the gel, and also the activity was associated with a second PAS-positive band that entered the gel. Some activity was found between the two major PAS-positive bands which entered the gel, probably indicating that variable quantities of the polysaccharides) did not migrate as a homogeneous band.

Fractionation and characterization of the polysaccharide in native ant venom

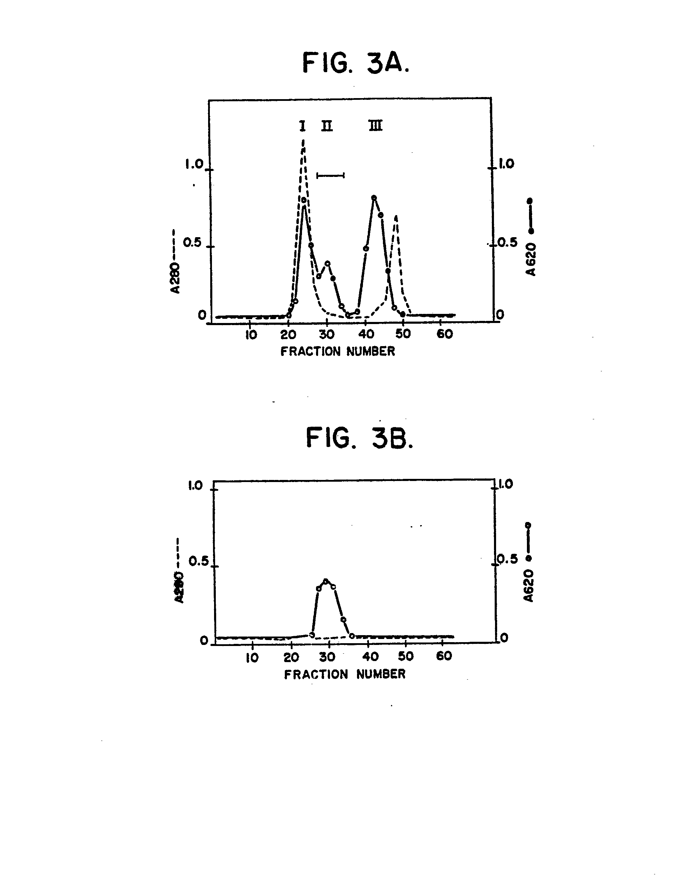

One-hundred milliliters of venom were concentrated to 2 ml by ultrafiltration with an Amicon UM-2 membrane (13.8 mg protein/ml) at 4°C and applied to a 1.1×70 cm column containing Sephadex® G-25 equilibrated with 5 mM sodium phosphate, 150 mM NaCl, pH 7.5, at 4°C. Individual fractions were assayed for protein by measuring absorbance at 280 nm, for sugars with anthrone reagent, and for the capability to cause consumption of serum C4 by functional hemolytic assays. For the latter, equal volumes of normal human serum and the column fraction were incubated for 4 hr at 37°C, and the titer of C4 was determined. The results in Fig. 3(A) show that 3 anthrone-positive peaks eluted, but only peaks I (PS-I) and II (PS-II) were analyzed further because peak III did not cause C4 consumption.

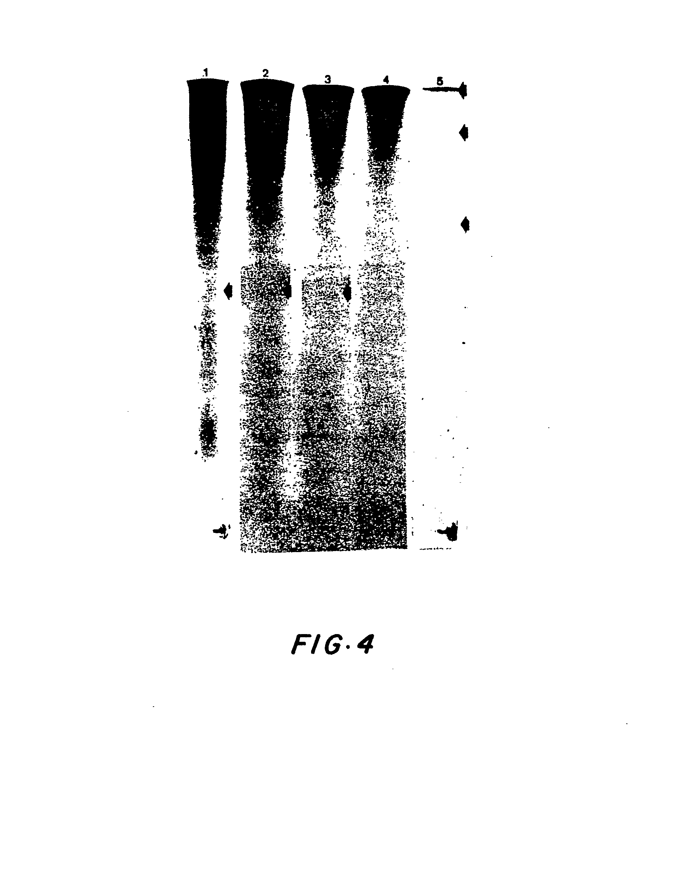

The anthrone-positive peaks PS-I and PS-II were pooled separately, concentrated by ultrafiltration (Amicon UM-2 membrane) at 4°C, and reapplied to the same column. Both peaks eluted in the same position as before. Thus, peak PS-II was significantly lighter than PS-I (Fig. 3B). The molecular weight was estimated to be 3000 daltons by the method of Andrews (1964). The individual fractions within the peaks were pooled, concentrated, and applied to polyacrylamide gels. Figure 4 (gel no. 1) shows that concentrated PS-I was heterogeneous, and individual bands were not distinguishable after staining the gel was PAS. Gels no. 2, 3, and 4 are concentrated PS-I diluted in saline 1:10, 1:20, and 1:40 respectively. Much of PS-I did not enter the gel or remained in the top portions, and in addition, a diffuse more anodal band is shown in gels no. 1, 2, and 3 (arrows). PS-II (gel no. 5) also was heterogeneous in the gel. A portion of the PS remained on the top of the gel, and 2 bands (arrows) were distinguishable within the gel.

Sugar composition of the venom PS

The concentrated fractions within peak PS-I (Fig. 3A) were analyzed for individual sugars by gas chromatography, and compared with the ethanol precipitate of the venom. Table 2 shows that 6 sugars were identified: mannose, N-acetylglucosamine, galactose, fucose, N-acetyl-galactosamine, and glucose. Mannose was present in the highest concentration, and glucose the least in concentration.

The alcohol precipitate of the venom, which had all of the polysaccharide and only a small portion of the total protein, also contained the same sugars by gas chromatographic analysis, and approximately the same molar ratios as PS-I. Since the protein(s) that remained after alcohol precipitation of the venom was anodal-migrating in polyacrylamide and did not stain with PAS (Fig. 2, gels no. 2 and no. 5), it is probable that the sugars that were measured by gas chromatography were from the PS and not from a glycoprotein(s).

In addition to the neutral sugars, a high content of hexuronic acid also was present in both PS-I and the alcohol precipitate, which explains the source of the net negative charge of the polysaccharide.

In other preliminary chemical assays with PS-I and the alcohol precipitate, they were not precipitated by barium salts, showing no apparent sulfate groups, and they were negative for phosphate content (Ames & Dubin, 1960).

IR spectra

An IR spectrograph was run on neat purified material. As would be expected, only three major peak were obtained. These are tabulated in Table 3.

Elemental analysis (CHO)

The purified material eluted from the Sepharose-Con A column and the alcohol precipitate (before purification in the Sepharose® -Con A column) from three separate lots of venom extract were sent to Galbraith Laboratories, Inc., Knoxville, Tenn, for elemental analysis. The results are tabulated in Table 4.

Solubility

To 3 ml of native ant venom (Lot 080678-WA) was added 27 ml of ice-cold absolute ethyl alcohol with stirring at 0°C to precipitate the active polysaccharide. A light precipitate formed, and the material was centrifuged in 6 tubes containing 5 ml each (International PR-2, 2000 RPM 10 min, 0°C). The ethanol was decanted, the six precipitates were dispersed at the bottom of each tube, and 5 ml of the following solvents were added: acetone, benzene, chloroform, anhydrous ethyl ether, propylene glycol, or glycerol. After mixing, the solubility of the precipitates was determined by visual inspection, at 25°C.

The results tabulated in Table 5 wherein insoluble means at least substantially insoluble; most of the material was apparently not dissolved. Soluble means most of the material appeared to be in solution.

TABLE 1

Consumption of C4 in normal human serum by eluates of polyacrylamide gel slices containing the isolated polysaccharide from ant venom (see Fig. 2). Equal volumes of each eluate and the serum were incubated at 37°C for 4 hr, and the C4 titer was determined by functional hemolytic assays.

| Migration distance | $CH_{50}$ units/ml of C4 in serum |
|---|---|
| 1—3[a] (Top) | 200 |
| 4—5 | 25,000 |
| 7—10 | 50,000 |
| 11—18 | 6,500 |
| 30—35 | 500,000 |
| 45—50 | 500,000 |
| 72—75 (Bottom) | 500,000 |
| Buffer Control | 500,000 |

[a]Contains both the PAS-staining material that did not enter the gels, and the first band within the gels.

TABLE 2

Sugar composition and the molar ratios of the native ant venom in Peak I (Fig. 3A) and the alcohol precipitate of the venom as determined by gas chromatography

Molar ratios

| Sugar | PI of G-25[a] (EtOH ppt.) | PI of G-25[a] (native) |
|---|---|---|
| Fucose | 1.5 | 1.8 |
| Mannose | 9.0 | 7.7 |
| Galactose | 1.1 | 1.4 |
| Glucose | 1.2 | 1.0 |
| N-acetyl glucosamine | 1.0 | 1.0 |
| N-acetyl galactosamine | 1.2 | 1.6 |

[a]Peak I from elution of Sephadex G-25 column (see text for details).

TABLE 3

Infrared

| Description of peak | Relative height (approx) [1]alcohol ppt | [2]man. eluate |
|---|---|---|
| Broad peak centered about 3460 cm$^{-1}$ | 8 | 5 |
| Broad peak centered about 2100 cm$^{-1}$ | 1 | 3 |
| Medium peak centered about 1640 cm$^{-1}$ | 5 | 2 |

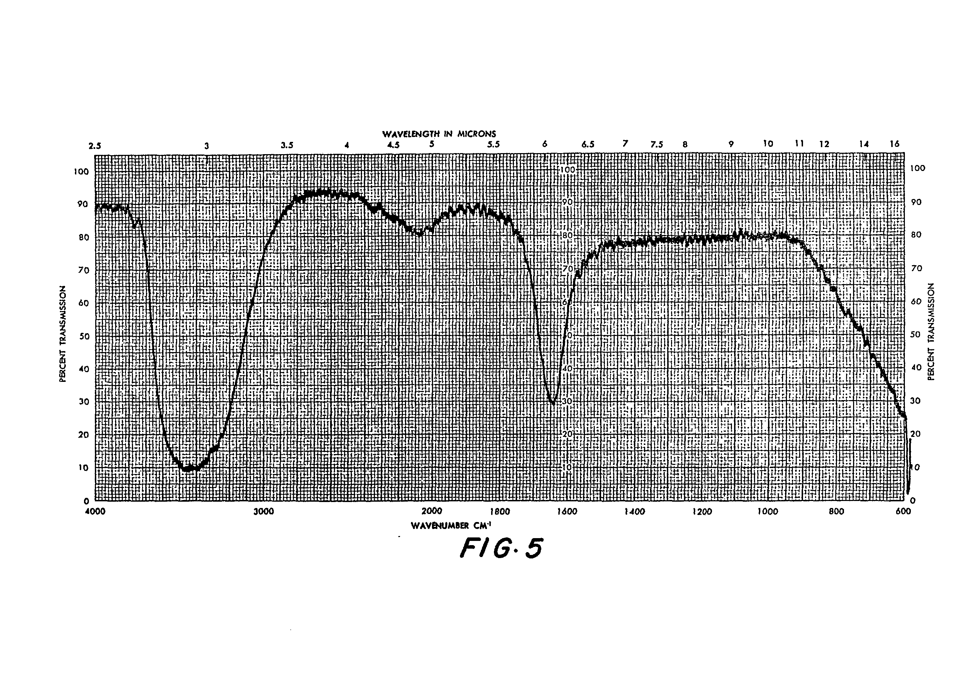

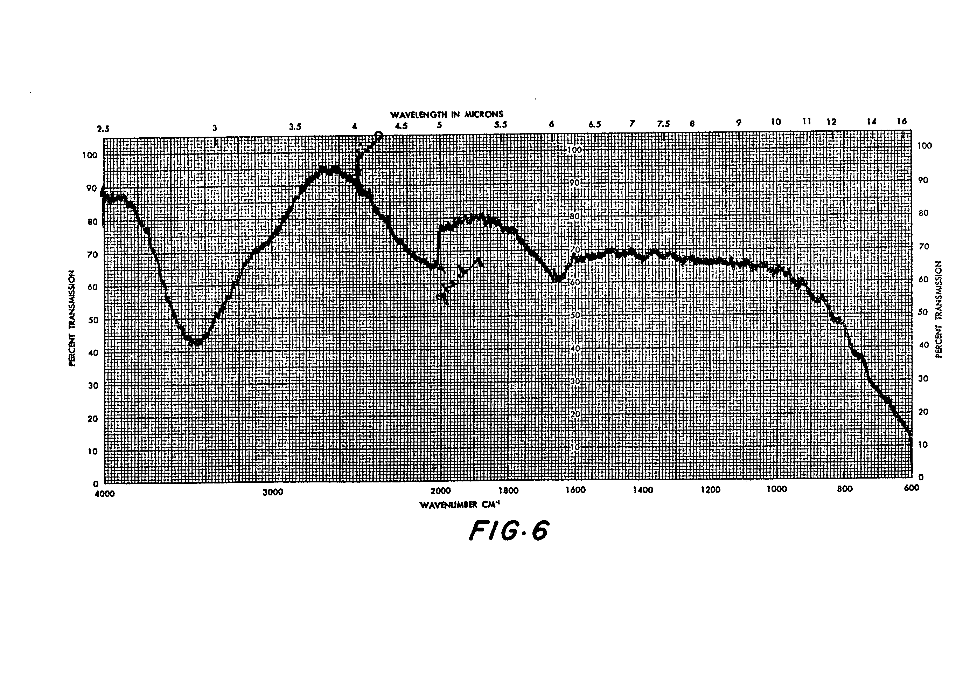

[1]Alcohol precipitate of ant venon from 500 ants (see Fig. 5)
[2]α D-Mannoside eluate of Con A column of alcohol precipitate (see Fig. 6)

TABLE 4

Elemental analysis

| Composition of sample | % C, | % H, | % O |
|---|---|---|---|
| EtOH ppt. of venom *lot 052778* | 41.19 | 6.08 | 37.62 |
| Same as 1, but Con a-Seph. eluate | 43.15 | 7.04 | 42.66 |
| EtOH ppt. of venom *lot 080678* | 37.15 | 5.81 | 35.75 |
| Same as 3, but Con A-Seph. eluate | 43.53 | 7.30 | 36.19 |
| EtOH ppt. of venom *lot 082678* | 37.70 | 6.50 | 35.60 |
| Same as 5, but Con A-Seph. eluate | 37.29 | 6.35 | 37.80 |

# 0 056 824

### TABLE 5
#### Solubility

| Solvent | Effect |
| --- | --- |
| Ethyl alcohol | Insoluble |
| Acetone | Insoluble |
| Benzene | Insoluble |
| Chloroform | Insoluble |
| Ethyl ether | Insoluble |
| Propylene glycol | Soluble |
| Glycerol | Soluble |
| Water | Soluble |
| Phosphate or acetate buffers in water, pH range 3 to 11 | Soluble |

## Claims

1. Composition for injection into a patient to provide remission of the symptoms of auto-immune ailments, characterized by a mixture of an aqueous extract of the venom sac of ants of the genus Pseudomyrmex specie triplarinus and a carrier compatible with said extract.

2. Composition of claim 1, characterized in that said mixture is extracted from the venom sac of said ants with saline physiological solution as the solvent.

3. Composition of claims 1 to 2, characterized in that said carrier is substantially a physiological solution.

4. Composition of claims 1, 2 or 3, characterized in that said ailment is rheumatoid arthritis.

5. Composition of any of claims 1 through 4, characterized by

a) a negative stain test for proteins with C. I. Acid Black 1, constitution no. 20470 (Amido Black 10B),

b) a positive stain test for glycoprotein and polysaccharide with periodic acid Schiff reagent (PAS), the combination of tests (a) and (b) leading to the conclusion that the composition is of a polysaccharide nature,

c) the consumption of $C_4$ (human blood complement system) upon incubation with human blood serum at 37°C for four hours,

d) a positive test for neutral sugars with anthrone reagent,

e) a positive test for hexuronic acids by carbazole method of Bitter and Muir,

f) not being precipitated from aqueous solution by barium salts, indicating the apparent absence of sulfate groups,

g) a negative phosphate test by the method of Ames and Dubin,

h) substantial solubility in propylene glycol, glycerol, water, phosphate buffers (aqueous) pH 3 to 11, and acetate buffers (aqueous) pH 3 to 11,

i) substantial insolubility in ethyl alcohol, acetone, benzene, chloroform and ethyl ether,

j) approximate sugar molar ratios:

| Sugar | Molar ratio range |
| --- | --- |
| Fucose | approximately 1—2 |
| Mannose | approximately 8—9 |
| Galactose | approximately 1—1,5 |
| Glucose | approximately 1 |
| N-acetyl glucosamine | approximately 1 |
| N-acetyl galactosamine | approximately 1—2 |

12

0 056 824

k) an approximate element analysis (CHO) of 35—45% carbon 6—7% hydrogen and 35—45% oxygen, and

l) an infrared spectrograph showing three major peaks approximately centered about 3460 cm$^{-1}$, 2100 cm$^{-1}$ and 1640 cm$^{-1}$.

6. Composition useful in the treatment of rheumatoid arthritis and other auto-immune aliments, characterized by

a) a negative stain test for proteins with C. I. Acid Black 1, constitution No. 20470 (Amido Black 10B),

b) a positive stain test for glycoprotein and polysaccharide with periodic acid Schiff reagent (PAS), the combination of tests (a) and (b) leading to the conclusion that the composition is of a polysaccharide nature,

c) the consumption of $C_4$ (human blood complement system) upon incubation with human blood serum at 37°C for four hours,

d) a positive test for neutral sugars with anthrone reagent,

e) a positive test for hexuronic acids by carbazole method of Bitter and Muir,

f) not being precipitated from aqueous solution by barium salts, indicating the apparent absence of sulfate groups,

g) a negative phosphate test by the method of Ames and Dubin,

h) substantial solubility in propylene glycol, glycerol, water, phosphate buffers (aqueous) pH 3 to 11, and acetate buffers (aqueous) pH 3 to 11,

i) substantial insolubility in ethyl alcohol, acetone, benzene, chloroform and ethyl ether,

j) approximate sugar molra ratios:

| Sugar | Molar ratio range |
| --- | --- |
| Fucose | approximately 1—2 |
| Mannose | approximately 8—9 |
| Galactose | approximately 1—1,5 |
| Glucose | approximately 1 |
| N-acetyl glucosamine | approximately 1 |
| N-acetyl-galactosamine | approximately 1—2 |

k) an approximate elemental analysis (CHO) of 35—45% carbon, 6—7% hydrogen and 35—43% oxygen, and

l) an infrared spectrograph showing three major peaks approximately centered about 3460 cm$^{-1}$, 2100 cm$^{-1}$ and 1640 cm$^{-1}$.

7. Method of preparing a composition effective to provide remission of symptoms of rheumatoid arthritis upon injection into a patient, characterized by

mixing the venom sacs of ants of the genus Pseudomyrmex specie triplarinus with saline physiological solution to form a mixture of insoluble parts and a solution of soluble parts of the any,

separating said solution from said mixture, and

adjusting the concentration of the solution to form said composition.

8. Method of claim 7, characterized in that said mixture is heated to about 37°C before said solution is separated from said mixture.

9. Method of claims 7 or 8, characterized in that the temperature of said solution is reduced to and held at a temperature below minus two degrees Celsius after separation from the mixture.

**Patentansprüche**

1. Injektionszusammensetzung zur Behandlung von Patienten gegen Symptome von Autoimmunleiden, gekennzeichnet, durch ein Gemisch aus einem wässrigen Extrakt aus dem Giftbeutel von Ameisen der Gattung Pseudomyrmex, Art triplarinus, und einem mit diesem Extrakt verträglichen Träger.

2. Injektionszusammensetzung nach Anspruch 1, dadurch gekennzeichnet, daß das Gemisch aus einem mit physiologischer Salzlösung als Lösungsmittel aus dem Giftbeutel der Ameisen gewonnenen Extrakt besteht.

3. Injektionszusammensetzung nach den Ansprüchen 1 oder 2, dadurch gekennzeichnet, daß der Träger aus physiologischer Lösung besteht.

4. Injektionszusammensetzung nach den Ansprüchen 1, 2 oder 3, dadurch gekennzeichnet, daß die Zusammensetzung eine solche zur Behandlung des Autoimmunleidens rheumatische Arthritis ist.

5. Injektionszusammensetzung nach irgend einem der Ansprüche 1 bis 4, gekennzeichnet durch

13

# 0 056 824

a) eine negative Färbereaktion bei Untersuchung auf Proteine mit C.I. Acid Black 1, 20470 (Amido Black 10B),

b) eine positive Färbereaktion bei Untersuchung auf Glykoprotein und Polysaccharid mit periodic acid Schiff-Reagenz (PAS), wobei die Kombination der Untersuchungen (a) und (b) zu dem Schluß führt, daß die Zusammensetzung ihrer Natur nach polysaccharidisch ist,

c) den Verbrauch von $C_4$ (Komplementsystem des menschlichen Bluts) bei 4-stündiger Inkubation mit menschlichem Blutserum bei 37°C,

d) eine positive Reaktion bei Untersuchung auf Neutralzucker mit Anthron-Reagenz,

e) eine positive Reaktion bei Untersuchung auf Hexuronsäuren mit der Carbazol-Methode nach Bitter und Muir,

f) fehlende Ausfällung mit Bariumsalzen aus wässriger Lösung, was die scheinbare Abwesenheit von Sulfatgruppen anzeigt,

g) eine negative Reaktion bei Untersuchung auf Phosphat mit der Methode nach Ames und Dubin,

h) wesentliche Löslichkeit in Propylenglykol, Glycerin, Wasser, (wässrigem) Phosphatpuffer bei pH 3 bis 11 und (wässrigem) Acetatpuffer bei pH 3 bis 11,

i) wesentliche Unlöslichkeit in Ethylalkohol, Aceton, Benzol, Chloroform und Ethyläther,

j) annähernd die folgenden Zuckerarten und deren relative molare Anteile:

| Zucker | Bereich des relativen molaren Anteils |
|---|---|
| Fruktose | annähernd 1—2 |
| Mannose | annähernd 8—9 |
| Galaktose | annähernd 1—1,5 |
| Glukose | annähernd 1 |
| N-Acetylglucosamin | annähernd 1 |
| N-Acetylgalaktosamin | annähernd 1—2, |

k) ein Elementaranalyse (CHO) von annähernd 35—45% Kohlenstoff, 6—7% Wasserstoff und 35—45% Sauerstoff, sowie

l) drei Haupt-Maxima im IR-Spektrum bei Wellenzahlen von annähernd 3460 cm$^{-1}$, 2100 cm$^{-1}$ und 1640 cm$^{-1}$.

6. Zur Behandlung von rheumatoider Arthritis und sonstigen Autoimmunleiden geeignete Mittelzusammensetzung, gekennzeichnet durch

a) eine negative Färbereaktion bei Untersuchung aus Proteine mit C.I. Acid Black 1, 20470 (Amido Black 10B),

b) eine positive Färbereaktion bei Untersuchung auf Glykoprotein und Polysaccharid mit periodic acid Schiff-Reagenz (PAS), wobei die Kombination der Untersuchungen (a) und (b) zu dem Schluß führt, daß die Zusammensetzung ihrer Natur nach polysaccharidisch ist,

c) den Verbrauch von $C_4$ (Komplementsystem des meschlichen Bluts) bei 4-stündiger Inkubation mit menschlichem Blutserum bei 37°C,

d) eine positive Reaktion bei Untersuchung auf Neutralzucker mit Anthron-Reagenz,

e) eine positive Reaktion bei Untersuchung auf Hexuronsäuren mit der Carbazol-Methode nach Bitter und Muir,

f) fehlende Ausfällung mit Bariumsalzen aus wässriger Lösung, was die scheinbare Abwesenheit von Sulfatgruppen anzeigt,

g) eine negative Reaktion bei Untersuchung auf Phosphat mit der Methode nach Ames und Dubin,

h) wesentliche Löslichkeit in Propylenglykol, Glycerin, Wasser, (wässrigem) Phosphatpuffer bei pH 3 bis 11 und (wässrigem) Acetatpuffer bei pH 3 bis 11,

i) wesentliche Unlöslichkeit in Ethylalkohol, Aceton, Benzol, Chloroform und Ethyläther,

j) annähernd die folgenden Zuckerarten und deren relative molare Anteile:

| Zucker | Bereich des relativen molaren Anteils |
|---|---|
| Fruktose | annähernd 1—2 |
| Mannose | annähernd 8—9 |
| Galaktose | annähernd 1—1,5 |
| Glukose | annähernd 1 |
| N-Acetylglucosamin | annähernd 1 |
| N-Acetylgalaktosamin | annähernd 1—2, |

k) eine Elementaranalyse (CHO) von annähernd 35—45% Kohlenstoff, 6—7% Wasserstoff und 35—45% Sauerstoff, sowie

l) drei Haupt-Maxima im IR-Spektrum bei Wellenzahlen von annähernd 3460 $cm^{-1}$, 2100 $cm^{-1}$ und 1640 $cm^{-1}$.

7. Verfahren zur Herstellung einer zur Injektion bei Patienten zwecks Behebung oder Verminderung von durch rheumatisch bedingte Arthritis verursachte Symptome geeignete Mittelzusammensetzung, dadurch gekennzeichnet, daß man Giftbeutel von Ameisen des Genus Pseudomyrmex, Spezies triplarinus, mit physiologischer Salzlösung vermengt und so ein Gemisch aus den unlöslichen Anteilen und einer Lösung der löslichen Anteile fertigt, die Lösung von dem Gemisch abtrennt, und

die Konzentration der Lösung einstellt und so die Zusammensetzung ausbildet.

8. Verfahren nach Anspruch 7, dadurch gekennzeichnet, daß man das Gemisch auf etwa 37°C erwärmt, bevor man die Lösung von dem Gemisch abtrennt.

9. Verfahren nach den Ansprüchen 7 oder 8, dadurch gekennzeichnet, daß man die Temperatur der Lösung erniedrigt und die Lösung auf einer Temperatur unterhalb −2°C hält, nachdem man die Lösung von dem Gemisch abgetrennt hat.

**Revendications**

1. Composition pour l'injection à un patient en vue d'obtenir la rémission des symptômes d'affections auto-immunitaires, caractérisée en ce qu'elle est un mélange d'un extrait aqueux du sac à venin de fourmis du genre Pseudomyrmex species triplarinus et d'un véhicule compatible avec cet extrait.

2. Composition suivant la revendication 1, caractérisée en ce que le mélange est extrait du sac à venin de ces fourmis au moyen de solution physiologique salée comme solvant.

3. Composition suivant la revendication 1 ou 2, caractérisée en ce que le véhicule est en substance une solution physiologique.

4. Composition suivant la revendication 1, 2 ou 3, caractérisée en ce que l'affection est l'arthrite rhumatoïde.

5. Composition suivant l'une quelconque des revendications 1 à 4, caractérisée par

a) un test coloré négatif pour les protéines avec le noir acide 1 du C.i. n° de constitution 20470 (noir amido 10B),

b) un test coloré positif pour les glycoprotéines et les polysaccharides avec le réactif à l'acide periodique de Schiff (PAS), la combinaison des tests (a) et (b) menant à la conclusion que la composition est de la nature des polysaccharides,

c) la consommation de $C_4$ (système de complément du sang humain) par incubation avec du sérum de sang humain à 37°C pendant 4 heures,

d) un test positif pour les sucres neutres avec le réactif à l'anthrone,

e) un test positif pour les acides hexuroniques par la méthode au carbazole de Bitter et Muir,

f) l'absence de précipitation à partir d'une solution aqueuse par les sels de baryum, indiquant l'absence apparente de radicaux sulfate,

g) un test au phosphate négatif suivant la méthode de Ames et de Dubin,

h) une solubilité sensible dans le propylène-glycol, le glycérol, l'eau, les tampons au phosphate (aqueux) de pH 3 à 11 et les tampons à l'acétate (aqueux) de pH 3 à 11,

i) une insolubilité sensible dans l'éthanol, l'acétone, le benzène, le chloroforme et l'éther éthylique,

j) les rapports molaires approximatifs entre sucres:

| Sucre | Intervalle de rapport molaire |
|---|---|
| Fucose | environ 1—2 |
| Mannose | environ 8—9 |
| Galactose | environ 1—1,5 |
| Glucose | environ 1 |
| N-Acétylglucosamine | environ 1 |
| K-Acétylgalactosamine | environ 1—2 |

k) une analyse élémentaire (CHO) approximative de 35—45% de carbone, 6—7% d'hydrogène et 35—45% d'oxygène, et

l) un spectrogramme infrarouge présentant trois pics majeurs centrés à peu près sur 3460 cm$^{-1}$, 2100 cm$^{-1}$ et 1640 cm$^{-1}$.

6. Composition utile pour le traitement de l'arthrite rhumatoïde et d'autres affections auto-immunitaires, caractérisée par

a) un test coloré négatif pour les protéines avec le noir acide 1 du C.I. n° de constitution 20470 (noir amido 10B),

b) un test coloré positif pour les glycoprotéines et les polysaccharides, avec le réactif à l'acide periodique de Schiff (PAS), la combinaison des tests (a) et (b) menant à la conclusion que la composition est de la nature des polysaccharides,

c) la consommation de C$_4$ (système de complément du sang humain) par incubation avec du sérum de sang humain à 37°C pendant 4 heures,

d) un test positif pour les sucres neutres avec le réactif à l'anthrone,

e) un test positif pour les acides hexuroniques par la méthode au carbazole de Bitter et Muir,

f) l'absence de précipitation à partir d'une solution aqueuse par les sels de baryum, indiquant l'absence apparente de radicaux sulfate,

g) un test au phosphate négatif suivant la méthode de Ames et du Dubin,

h) une solubilité sensible dans le propylène-glycol, le glycérol, l'eau, les tampons au phosphate (aqueux) de pH 3 à 11 et les tampons à l'acétate (aqueux) de pH 3 à 11,

i) une insolubilité sensible dans l'éthanol, l'acétone, le benzène, le chloroforme et l'éther éthylique,

j) les rapports molaires approximatifs entre sucres:

| Sucre | Intervalle de rapport molaire |
|---|---|
| Fucose | environ 1—2 |
| Mannose | environ 8—9 |
| Galactose | environ 1—1,5 |
| Glucose | environ 1 |
| N-Acétylglucosamine | environ 1 |
| N-Acétylgalactosamine | environ 1—2 |

k) une analyse élémentaire (CHO) approximative de 35—45% de carbone, 6—7% d'hydrogène et 35—45% d'oxygène, et

l) un spectrogramme infrarouge présentant trois pics majeurs centrés à peu près sur 3460 cm$^{-1}$, 2100 cm$^{-1}$ et 1640 cm$^{-1}$.

7. Procédé de préparation d'une composition efficace pour assurer la rémission de symptômes de l'arthrite rhumatoïde par injection à un patient, caractérisé par

le mélange des sacs à venin de fourmis du genre Pseudomyrmex species triplarinus avec de la solution physiologique salée pour former un mélange de parties insolubles et d'une solution de parties solubles,

la séparation entre la solution et le mélange, et

l'ajustement de la concentration de la solution pour former la composition.

**0 056 824**

8. Procédé suivant la revendication 7, caractérisé en ce que le mélange est chauffé à environ 37°C avant que la solution soit séparée du mélange.

9. Procédé suivant la revendication 7 ou 8, caractérisé en ce que la température de la solution est abaissée et maintenue à une valeur inférieure à moins deux degrés Celsius après la séparation hors du mélange.

# FIG. I.

PURIFICATION OF POLYSACCHARIDE FROM NATIVE ANT VENOM

ANT VENOM (A.V.)

↓

9 PARTS ABSOLUTE ETHANOL (0°C) TO I PART A.V.

STIR, 0°C, FOR I HOUR

CENTRIFUGE

PRECIPITATE           SUPERNATANT ⟶ DISCARD

↓

WASH IX, 90% ETHANOL, 0°C.

SUSPEND IN DISTILLED WATER

LYOPHILIZE

↓

SUSPEND IN DISTILLED WATER

CENTRIFUGE

↓

DIALYZE SUPERNATANT, 0.018M NaPO$_4$ IN 0.15M NaCl, pH 7.2 (PBS)

↓

COLUMN: CONCANAVALIN A-SEPHAROSE; BUFFER, PBS

↓

WASH WITH 0.3M BORATE BUFFER, pH 8.7

↓

ELUTE WITH 0.1M α-METHYL –D–MANNOSIDE IN PBS

↓

PURIFIED POLYSACCHARIDE

FIG.2

# FIG. 3A.

# FIG. 3B.

*FIG.4*

FIG·5

FIG·6

0 056 824